# EUROPEAN PATENT APPLICATION

(11) **EP 3 263 156 A1**
(43) Date of publication of application: **03.01.2018**
(21) Application number: 16177341.1
(22) Date of filing: 30.06.2016
(51) Int. Cl.: A61M 5/145, A61M 5/142, A61M 5/36, A61B 1/06, G02B 17/00, G01N 21/51, G01N 21/53, F21V 8/00

(54) **OPTICAL WAVEGUIDE RESERVOIR ILLUMINATION FOR A LIQUID DRUG DELIVERY DEVICE**

(71) Applicant: Roche Diabetes Care GmbH, 68305 Mannheim (DE); F. Hoffmann-La Roche AG, 4070 Basel (CH)
(72) Inventor: The designation of the inventor has not yet been filed
(74) Representative: Richardt Patentanwälte PartG mbB

(57) **Abstract**

The invention provides for a drug delivery device (130), comprising a hollow container comprising a wall structure and an internal volume (110) surrounded by the wall structure, the hollow container being adapted to contain within or transport through the internal volume a liquid drug to be supplied to a patient, the wall structure comprising an optical waveguide wall material (106), the optical waveguide wall material comprising a light scattering characteristic; and a light source (120), adapted to illuminate the internal volume by emitting illumination light such that the illumination light is coupled into the optical waveguide wall material through a light coupling area (122) of the wall structure, propagates through the optical waveguide wall material by total internal reflection and is scattered into the internal volume by the light scattering characteristic.

## Description

### Field of the invention

The present invention relates to medical devices performing the delivery of liquid drugs, in particular to a method for illuminating parts carrying the liquid drug.

### Background and related art

Devices and systems for the delivery of liquid drugs are widespread throughout nearly all fields of health care. They usually contain the liquid drug to be supplied to a patient within a reservoir and possess a mechanism to deliver the drug to the patient by means of a hollow needle (cannula) and, optionally, a tubing (hose) interfacing a reservoir outlet and the cannula. The drug delivery may be a manual process (as with a syringe) or be controlled by an automatic system (such as electrically driven programmable pumps). Mobile and stationary devices are employed depending on the specific care situation.

Illuminating the reservoir of a liquid drug delivery system is especially interesting for scenarios where the device must be used throughout the night or in other dark environments regularly. Full independence from external light sources is possible for devices where an integrated illumination is possible. A widely used example is given by mobile insulin pumps, as they are commonly deployed in continuous subcutaneous insulin infusion therapy. Such devices usually include a pumping apparatus, control units and user interfaces in addition and are connected to an infusion set ensuring a firm placement of the cannula typically for two days. Whereas the examples presented in the following are mostly restricted to insulin pumps, it is understood that the disclosed invention may be applied to nearly all other kinds of drug delivery devices comprising transparent hollow containers adapted to carry a liquid drug to be supplied to the patient.

According to the current state of the art, optical inspection of a liquid drug reservoir can be aided by local illumination, where small light sources are used to shine through a transparent reservoir, allowing the user to examine the transmission characteristics of the liquid drug. In mobile systems this is a consequence of the need for high integration and miniaturization. In United States patent application US 2009/0216194 A1 a drug delivery device reservoir illumination system is disclosed, wherein light conductors are used to determine the optical transmission through a portion of the reservoir. Moreover, an illuminator assembly optimized for use with suspended reservoir bags and drip components for IV drug delivery is shown in United States patent application US 2004/0160770 A1.

### Invention summary

The present invention relates to a drug delivery device comprising a hollow container and a light source, the hollow container comprising a wall structure and an internal volume surrounded by the wall structure, the hollow container being adapted to contain within or transport through the internal volume a liquid drug to be supplied to a patient, a wall structure comprising an optical waveguide wall material, the optical waveguide wall material comprising a light scattering characteristic; the light source being adapted to illuminate the internal volume by emitting illumination light such that the illumination light is coupled into the optical waveguide wall material through a light coupling area of the wall structure, propagate through the optical waveguide wall material by total internal reflection and scattered into the internal volume by the light scattering characteristic.

As used herein, a liquid drug may be any liquid supplied to a patient during medical therapy or any other kind of healthcare treatments, including, but not limited to, pure liquid substances comprising one fluid component; liquid solutions comprising a dissolved agent; liquid suspensions of molecules, droplets, or particles; as well as any other type of liquid mixtures, wherein all preparations of insulin or insulin analogues, as they are used in insulin infusion therapy, are expressly included. A drug is further understood as any kind of medication such as antibiotics, chemotherapy drugs, pain relievers, insulin etc.

A hollow container as used herein may be any part adapted to contain or transport a liquid drug within an internal volume, such as liquid drug reservoirs, hoses, fluid system connectors, cannulas, or any other kind of tubing comprising an optical waveguide wall material, which in turn may be a synthetic material such as polyethylene, glass, or any other transparent material allowing for light propagating through said material by total internal reflection. Independent from its choice, an optical waveguide wall material will always possess an intrinsic ability to scatter light propagating through the optical waveguide material. However, this ability is usually a merely weak effect, meaning that the intensity of the scattered light is small compared to the intensity of the light which remains propagating through the optical waveguide material.

For the purpose of illuminating the internal volume of a hollow container, it may therefore be useful to add a further feature to the optical waveguide material which is adapted to enhance the light scattering ability of the material. This intrinsic property of the optical waveguide material to scatter light as well as any added feature enhancing the intrinsic light scattering ability, including light scattering particles embedded into the optical waveguide material or a light scattering structure of a wall structure surface, shall individually be referred to as a light scattering characteristic comprised by the optical waveguide material.

The chosen frequency range for the illumination light depends on how the light emerging the illuminated hollow container is processed. Visible light is deemed to be the range of choice for most applications, especially if the illuminated hollow container is to be inspected directly by the user. However, one may also think of automated usage scenarios where the illuminated liquid drug is optically inspected by an optical detector and an inspection by the user is not needed. In such cases it may be advantageous to use a light source in a different wavelength range, such as an infrared light source, instead.

The advantages of the described drug delivery device may arise from the fact that the hollow container is used as an optical waveguide. Typical shortcomings of present illumination solutions for liquid drug delivery devices may include a mere local illumination of the reservoir; non-obvious scalability towards an optimized integrated design for mobile devices, and a lack of applicability for an illumination of liquid drug tubings with a high brightness along their full length. In contrast, the present invention may allow for an illumination of the whole container instead of only a portion of it, while the light source needed to illuminate the large volume may be compact. With the hollow container being illuminated as a whole, a more reliable inspection of its content, e.g., for air bubbles, the reservoir fill level, or indications of insulin fibrillation, and/or of the wall material condition, e.g., for damages such as punctures, may be possible. Furthermore, the light guide principle may provide a very efficient method to provide light to the interior of the container. It may also allow for the use of only one central light source, which may be detached from a region of interest where the illumination is needed.

Air bubbles that are captured in the parts carrying the liquid drug may not be beneficial since an insertion of air into the patient's body should be avoided especially for applications involving intravenous (IV) drug delivery. Furthermore, an inspection of the fill level may be desirable, especially in automated usage scenarios where the liquid drug is not delivered at a constant rate. Checking illuminated device parts for air bubbles and determining the reservoir fill level may be done by the user or as an automatic function of the delivery system.

A peculiarity concerning insulin pumps is the ability of the dissolved insulin molecules to polymerize under certain conditions regarding temperature and / or material properties of the parts in contact with the insulin solution. This process (commonly called "fibrillation") inhibits the bodily effect of insulin to a large extent. If fibrillation does not cause a congestion of the tubing and / or cannula, a timely detection of fibrillation within a primed delivery system is usually difficult, as there are often no further signs of fibrillation than tiny layers of deposit which become clearly visible only in optically transparent parts if brought in front of a bright light source or if the insulin is removed from the primed parts. A bright, complete and homogeneous illumination of all parts with insulin contact, especially those with a narrow cross section such as tubings, cannulas and connectors, could greatly improve fibrillation inspection for diabetes patients. The invention may allow for a respective illumination of the container in order to efficiently detect by a user or automatically by optical inspection by the drug delivery device signs of fibrillation.

In accordance with one embodiment of the invention, the drug delivery device further comprises an optical coupling element adapted to couple the illumination light through the light coupling area into the optical waveguide wall material. The optical coupling element serves the purpose to improve the coupling efficiency of the light to be coupled into the optical waveguide material, and to customize the propagation characteristics of the illumination light to the requirements of the respective application scenario. Optical coupling elements may be chosen from a large variety of known optical elements such as lenses, reflective surfaces, impedance couplers, fiber optical parts, etc. As an example, in another embodiment, the hollow container is an injection molded part and the optical coupling element is adapted to couple the illumination light into the optical waveguide wall material such that the propagation direction of the illumination light entering the optical waveguide wall material is parallel to the draw direction of the injection molded hollow container. Thus, the optical coupling is performed in a manner that the light propagating through the optical waveguide material experiences a minimal attenuation otherwise caused by the light having to cross molecular structures in the draw direction.

Hollow containers used for liquid drug delivery devices usually are of an oblong shape such that longitudinal and radial directions can be defined. According to another embodiment of the invention, the light coupling area is parallel to a cross-sectional wall area of the wall structure, the cross-sectional wall area being defined with respect to a radial extension of the internal volume. A light coupling area thus oriented may further improve the coupling of illumination light in a direction favorable for total internal reflection in the optical waveguide material. The optical coupling element may also be utilized to distribute the light to be coupled into the optical waveguide material homogeneously across a larger light coupling area.

According to another embodiment, the light coupling area covers the whole cross-sectional wall area, the optical coupling element being adapted to couple the illumination light into the optical waveguide wall material in a manner which minimizes the spatial light intensity variation across the light coupling area. This may have the advantage that a homogeneous illumination of the whole hollow container can be achieved, further improving a comprehensive inspection of the internal volume.

An optical coupling element may also be a light guide part being adapted to a particular shape of the wall structure cross section. Such part may, e.g., be produced from a synthetic material, which may be easily brought into a shape that matches a particular geometry of the hollow container.

In accordance with a further embodiment, the hollow container is e.g. of a cylindrical shape and, in general, both the cross-sectional wall area and the optical coupling element have an annular shape. Alternatively or additionally, the optical coupling element may comprise smaller sub-structures serving as individual optical coupling elements, which shall be called optical coupling segments. In another embodiment of the invention, the drug delivery device further comprises multiple ones of the light source, the optical coupling element comprising multiple coupling segments, and each coupling segment being optically connected to at least one of the light sources and the light coupling area. This may be used as an effective means to generate a homogeneous illumination of the hollow container. Optionally, at least one of the coupling segments may be interconnecting at least two of the light sources to increase the amount of optical energy per light source coupled into the optical waveguide material.

In accordance with an embodiment of the invention, the light scattering characteristic is an intrinsic property of the optical waveguide wall material and/or light scattering particles embedded into the optical waveguide wall material and/or a light scattering structure of a surface of the wall structure.

In one embodiment of the invention, the optical waveguide wall material further comprises a light scattering region, wherein the light scattering characteristic is localized in a light scattering region of the optical waveguide wall material, the light scattering characteristic being distributed within the light scattering region according to a local scattering density, which is a function of the position within the light scattering region. This may allow for illumination only a defined and limited region of interest of the container, complementing the illumination of the whole hollow container. It is to be understood that the container may comprise more than one regions of interest. Nevertheless, instead of using one light source per region of interest embodiments may provide for various light scattering regions spaced apart from each other and adapted to scatter the light e.g. emanated from a single light source to respective regions of interest.

In another embodiment, the local scattering density may be a function of a spatial coordinate in the propagation direction of the illumination light within the optical waveguide wall material. This may allow for using the light scattering characteristic as a means to compensate the attenuation of the light propagating along the optical waveguide material, which may be especially useful for hollow container structures spanning a large longitudinal distance, such as a tubing interfacing an insulin pump reservoir and a connector or a cannula of an infusion set. The attenuation, which is usually described by a decreasing exponential function with a constant attenuation coefficient, may be compensated by arranging the light scattering characteristic according to a local scattering density being an increasing linear function of the position in an average longitudinal propagation direction of the light inside the optical waveguide material.

In accordance with another embodiment, the hollow container further comprises a supporting structure, the supporting structure comprising an optical waveguide support material and being optically connected to the optical waveguide wall material , the light source and/or the optical coupling element being adapted to couple the illumination light through a light coupling area of the supporting structure such that the illumination light propagates through the optical waveguide support material and the optical waveguide wall material by total internal reflection. This approach may combine solutions addressing the need for a mechanical supporting structure for the hollow container and for maximizing the optical energy being coupled into the optical waveguide wall material.

The supporting structure may be manufactured together with the rest of the hollow container in the same production step, as it is the case in an injection molding process, for instance, and therefore possess a very efficient optical connection to the optical waveguide wall material, while offering a larger surface area for coupling the illumination light into the optical waveguide material.

In accordance with an embodiment of the invention, the light coupling area is parallel to an intersectional area of the supporting structure and a cross-sectional wall area of the wall structure, the cross-sectional wall area being defined with respect to a radial extension of the internal volume. For example, intersectional areas between the supporting structure and the cross-sectional wall area of the wall structure may exist, the cross-sectional wall area being once more defined with respect to a radial extension of the internal volume, where the coupling loss for light coupled into the optical waveguide support material is especially low, enabling a highly efficient coupling of the illumination light into the optical waveguide wall material.

In another embodiment, the hollow container is a reservoir adapted to contain the liquid drug.

In accordance with a further embodiment of the invention, the hollow container further comprises a drug outlet and a tubing, the drug outlet being in fluid connection to the internal volume, the tubing comprising an internal tubing volume and an optical waveguide tubing material surrounding the internal tubing volume, the tubing being adapted for extending the fluid connection from the drug outlet through the internal tubing volume to a connector or cannula of an infusion set adapted to supply the liquid drug to the patient, the light coupling area being a cross-sectional area of the optical waveguide tubing material with respect to a radial extension of the tubing. According to the latter aspect, an illumination of the whole tubing may become possible, especially if light scattering characteristics as described before are used to overcome the attenuation encountered by the illumination light propagating through the tubing. The illumination light transported by the tubing may be coupled even further into a transparent connector of an infusion set and/or a transparent cannula, as they are often used during insulin infusion therapy. This may allow for an inspection of the inner wall surfaces of said connector and/or cannula for films of fibrillated insulin or possible sources of obstruction.

It has to be noted that for example the drug outlet may be sealed by a septum such that the septum is located between the drug outlet and the internal volume.

In accordance with a further embodiment of the invention, the internal volume is filled with a liquid drug to be supplied to the patient.

In another aspect, the invention relates to a drug delivery system comprising a drug delivery device according to any of the embodiments described before, the hollow container further comprising a drug outlet being in fluid connection to the internal volume. The drug delivery system further comprises a pumping apparatus adapted for supplying the liquid drug to the patient by pressing the liquid drug through the drug outlet. The drug delivery system further comprises a light control unit adapted for generating an electrical control signal to be transmitted to the light source.

For example, drug delivery devices according to any of the aspects or embodiments described before are part of the drug delivery system. A drug delivery system may also comprise a pump control unit and various user interface functions, allowing for an automated supply of the liquid drug according to a programmable time profile.

In one embodiment, the invention provides for a method of illuminating the internal volume of a drug delivery system described before e.g. illuminating the interior of the hollow container, the method comprising coupling the illumination light into the optical waveguide wall material such that the illumination light propagates through the optical waveguide wall material by total internal reflection and is scattered into the internal volume by the light scattering characteristics. This method may utilize the prospective advantages provided by any of the drug delivery devices and/or a drug delivery system as described before.

In another embodiment, the method further comprises optically inspecting the internal volume for gas bubbles, and in another embodiment, the method further comprises optically determining the fill level of the liquid drug contained within the internal volume. The method according to each of the aforementioned embodiments may be performed by an electronic system adapted to analyze the output of an optical detector in terms of, for instance, the transmission properties of the internal volume.

In a further embodiment, said method comprises modulating the light source by the light control unit for transmission of information to a user, the information being a status indication of the drug delivery device and/or system.

The status indication is understood as any information regarding the operational state of the drug delivery device and/or system. The information includes e.g. the fill level of the drug in the reservoir, the presence of air bubbles in the drug, the state of the power source used to electrically power the system, occlusion etc,.

The particular method for modulating the light source may be chosen from a multitude of well-known modulation techniques. As an example, the electrical control unit may be used to send a pulse signal to the light source such that the user is informed by a blinking illumination pattern of the hollow container. One may also think of a signal wherein the voltage is varied in a step or continuously, such that the user is informed by a sudden or gradual change of illumination intensity of the hollow container. Furthermore, one may think of changing the color of the light source by using a multichannel signal.

The method according to this embodiment may be executed on the basis of an analysis result obtained from an automated optical inspection as described before, or on equivalent status information obtained by different methods including a non-optical status check. An exemplary application scenario involving the method according to all embodiments mentioned before may be a self-test program of the drug delivery system involving, as a first step, illuminating the internal volume with illumination light scattered out of the optical waveguide wall material, and, as a second step, optically inspecting the internal volume for gas bubbles. If gas bubbles are detected, the illumination color may for example be changed to blue and the illumination is set into continuous blinking mode. If no gas bubbles are detected, the liquid drug fill level of the internal volume is optically determined as a third step. If the fill level is detected to be below e.g. 5% of a full charge of the hollow container, the illumination color may be changed to red and the illumination is set into continuous blinking mode. If the fill level is not below e.g. 5% of a full charge, the illumination color may be changed to green and gradually switched off after 30 seconds of continuous illumination.

It is understood that one or more of the aforementioned embodiments of the invention may be combined as long as the combined embodiments are not mutually exclusive.

### Short description of the figures

In the following, embodiments of the invention are explained in greater detail, by way of example only, making reference to the drawings in which:
- Fig. 1: shows an exemplary drug delivery system;
- Fig. 2: shows a light source attached to a reservoir in an enlarged section of Fig. 1;
- Fig. 3: shows a top view of a hollow container with a supporting structure and light sources being installed on the supporting structure;
- Fig. 4: shows a top view of a hollow container with a light source and an optical coupling element;
- Fig. 5: shows a top view of a hollow container with multiple light sources and an optical coupling element comprising multiple coupling segments;
- Fig. 6: shows a light source attached to a hollow container wall structure comprising light scattering particles;
- Fig. 7: shows a variant of Fig. 6, the light scattering particles being localized in a light scattering region;
- Fig. 8: shows a usage scenario of an insulin pump; and
- Fig. 9: shows a flowchart of an exemplary self-test routine of a liquid drug delivery system.

### Detailed description

Like numbered elements in these Figs. are either equivalent elements or perform the same function. Elements which have been discussed previously will not necessarily be discussed in later Figs. if the function is equivalent.

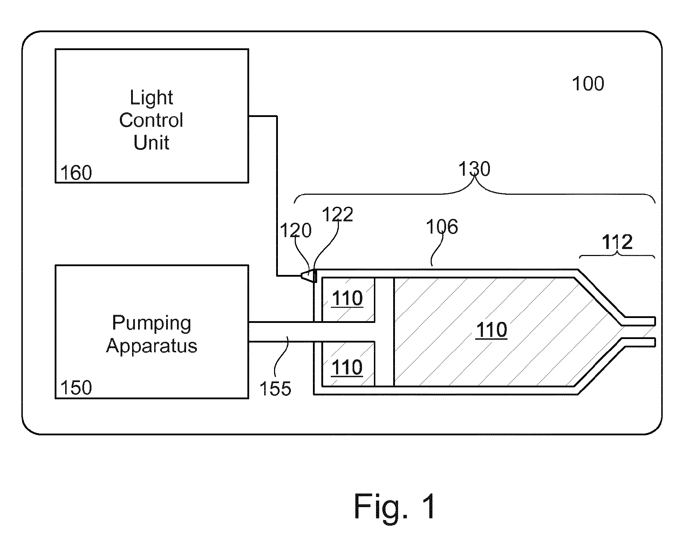

Fig. 1 shows a schematic view of an exemplary embodiment of a liquid drug delivery system 100, comprising a liquid drug delivery device 130, a pumping apparatus 150 and a light control unit 160. The drug delivery device 130 comprises a hollow container 106 and a light source 120 optically coupled to an optical waveguide wall material 106 comprised by the wall structure, which further comprises a drug outlet 112. The internal volume 110 of the hollow container may contain a liquid drug to be supplied to a patient, shown as a hatched area. In the example shown, this is done by the pumping apparatus 150, which comprises a piston 155 inserted into the internal volume 110. During use, the piston 155 is pushed longitudinally towards the drug outlet 112, thus expelling the liquid drug through the drug outlet 112 and supplying the drug to the patient. The illumination light 600 emitted by the light source 120 is coupled through a light coupling area 122 into the optical waveguide wall material 106 of the wall structure in a manner allowing the light to proceed through the optical waveguide wall material 106 by total internal reflection. The light scattering characteristic of the optical waveguide wall material 106 leads to a scattering of the light travelling through the material 106. Due to the scattering, the liquid drug is illuminated by the illumination light.

The light source 120 is electrically connected to a light control unit 160 adapted to modulate the light source 120, for example, by switching it on or off, changing the light intensity, or changing the color of the illumination light 600. Any light source 120 suitable for being integrated into a compact drug delivery system 100 may be chosen for the proposed illumination scheme, but a light source 120 based on LED technology may be preferable due to the greatly reduced heat generation, improved scalability compared to light bulbs, and enhanced integratability for applications involving changing light colors. Using the wall structure of the hollow container as an optical waveguide is intended to enable an illumination of the whole hollow container or a portion of it. This is to be achieved by an optimized coupling of the illumination light 600 into the optical waveguide wall material 106, which may be accomplished by an appropriate choice of the light coupling area 122.

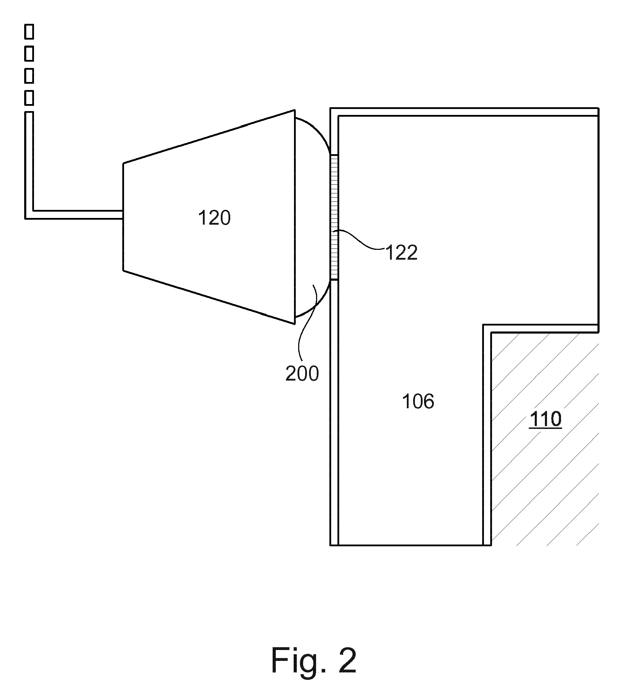

Fig. 2 shows an enlarged section of Fig. 1 focusing on the light source 120 and the light coupling area 122. In addition to Fig. 1, an optical coupling element 200 is interfacing the light source 120 and the light coupling area 122 here. An optical coupling element 200 may be any structure capable of improving the optical connection between the light source 120 and the optical waveguide wall material 106, i.e., increasing the optical power transported from the light source 120 into the optical waveguide wall material 106. Optical coupling elements may be chosen from a broad range of optical parts or structures with optical properties, such as lenses, gratings, mirrors or reflecting surfaces, fiber optical components, or impedance couplers.

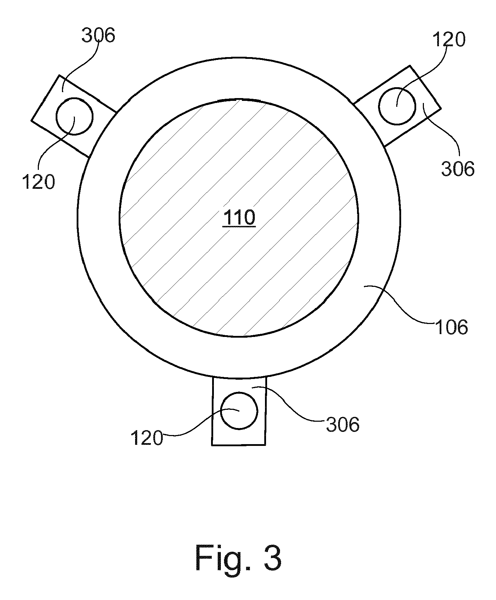

Fig. 3 shows a top view of a hollow container with a circular cross section, mechanically supported by three segments of the supporting structure comprising an optical waveguide support material 306. One light source 120 is mounted on each supporting structure segment. This arrangement may be advantageous if the supporting structure offers a larger area suitable for coupling the illumination light 600 into the wall structure in the desired manner, and/or if the supporting structure possesses a good optical connection to the optical waveguide wall material 106 by construction. This may be the case if the supporting structure is produced together with the wall structure in the same step, such as in an injection molding process.

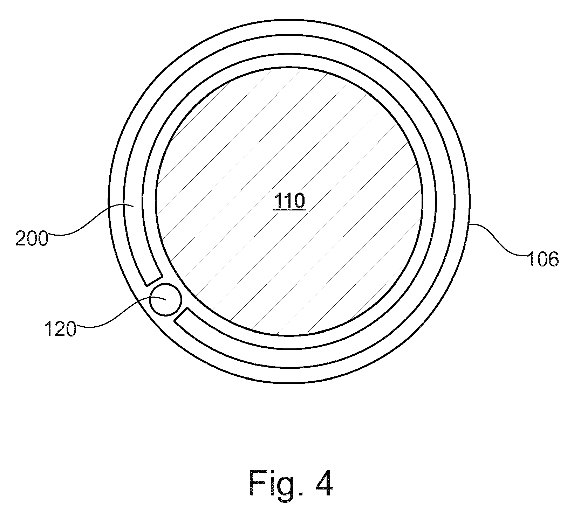

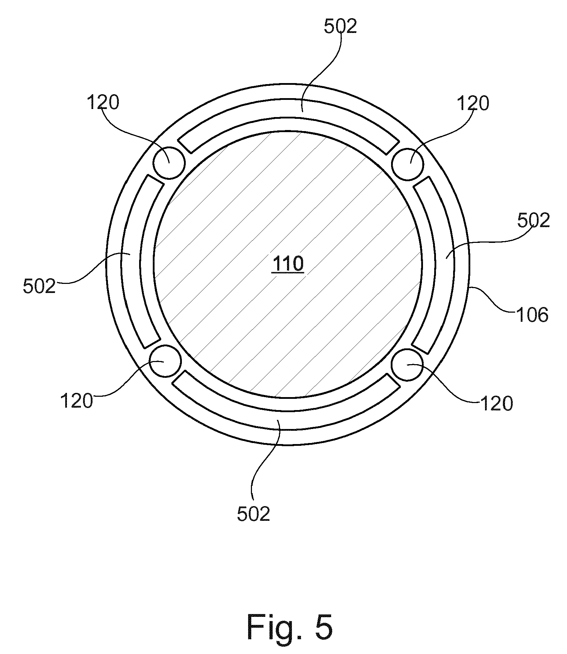

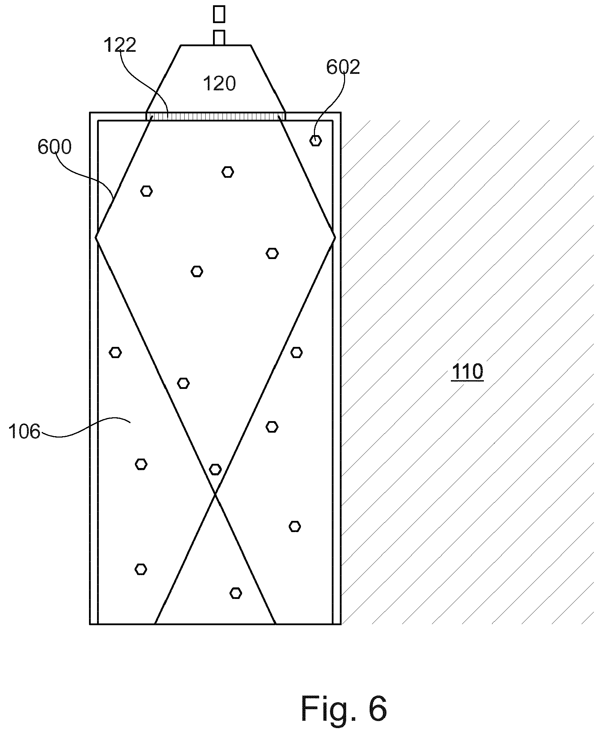

Referring to Fig. 4, a top view of a hollow container with a circular cross section is shown, a light source 120 being located on top of the cross section of the wall structure, together with an annular optical coupling element 200. According to this example, a more homogeneous illumination of the hollow container may be achieved by an optical coupling element 200 adapted to the cross-sectional geometry of the hollow container, which may be easily manufactured from a synthetic material. The annular optical coupling element 200 may be equipped with a light scattering characteristic to direct an illumination light 600 emerging the light source 120 into the optical waveguide wall material 106 (compare Fig. 6). The coupling efficiency may be further enhanced by manufacturing the coupling element 200 with a transparent planar side facing the hollow container, and a reflective curved side opposite of the planar side. The reflectivity of the curved side may be enhanced by a surface treatment such as coating the curved side with a thin layer of a reflective material or a material with a lower refractive index than that of the optical coupling element 200. As shown in Fig. 5, an optical coupling element 200 may be divided into multiple optical coupling segments 502 if multiple light sources 120 are used. This may also lead to a homogeneous illumination of the hollow container, while increasing the illumination intensity.

Fig. 6 shows a cross section of a part of the wall structure, the optical waveguide wall material 106 being equipped with light scattering particles 602, and illumination light 600 being coupled into the optical waveguide wall material 106. Whereas the ability to scatter light is an intrinsic property of all materials suitable to serve as an optical waveguide wall material, this intrinsic light scattering characteristic may be too weak to generate a sufficient illumination intensity of the internal volume 110 for the purposes described before. Hence, it may be advantageous to add further light scattering characteristics to the optical waveguide wall material 106. As suggested in the Figure, this may be accomplished by light scattering particles 602, but one may think of other means to improve the light scattering into the internal volume 110, such as roughening or engraving the outer surface of the wall structure. Embedding light scattering particles 602 into a synthetic optical waveguide wall material 106 may be accomplished by adding an additive to the raw material during the production process of the synthetic wall material. Suitable additives may be metallic dust grains or any other reflective particles which are stable under the thermal conditions encountered during the formation of the reflective optical waveguide wall material 106.

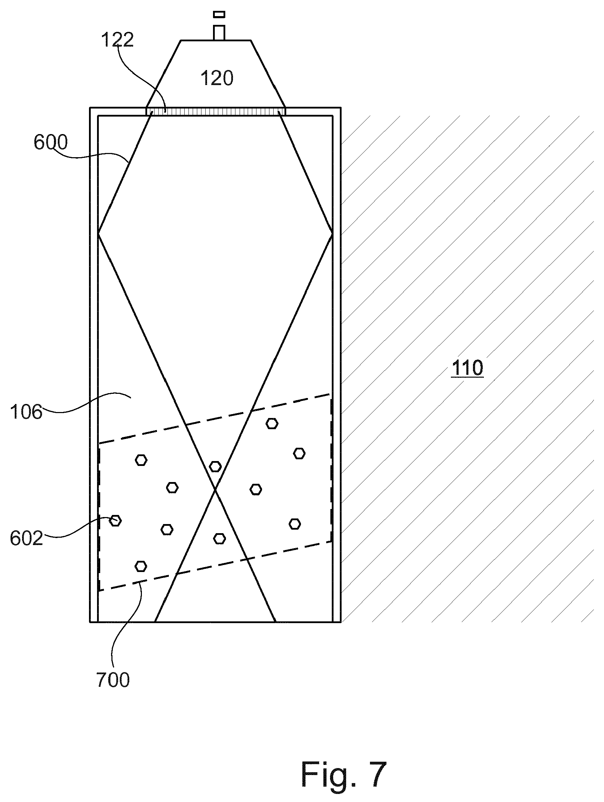

Fig. 7 shows an alternative embodiment to the one shown in Fig. 6, with the difference that the light scattering particles 602 are localized within the light scattering region 700 indicated by a dashed line. This variant may be advantageous if further regions of interest exist besides the hollow container. For example, one may think of a drug delivery system 100 comprising a hollow container in any of the senses used before, and parts of the user interface, such as a display or buttons, for which an illumination alongside the hollow container may be desirable. For this scenario, the wall structure may be extended towards the further parts to be illuminated, the illumination light 600 being transported through the whole optical waveguide wall material 106, but being scattered only at the hollow container and the user interface parts to be illuminated.

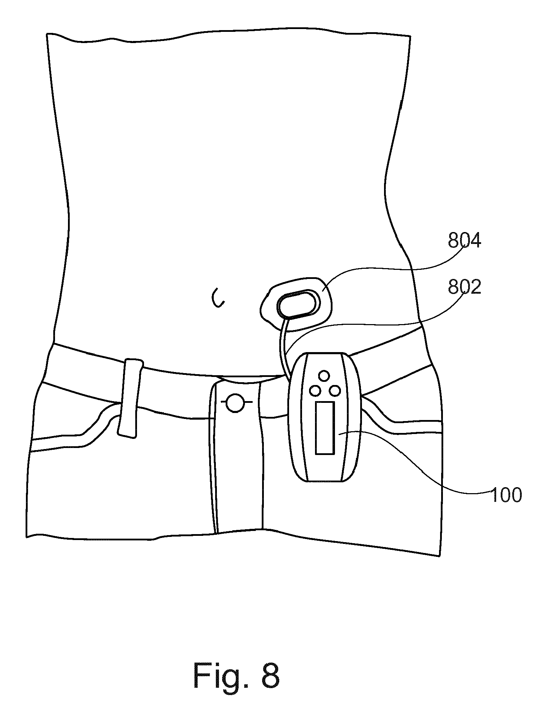

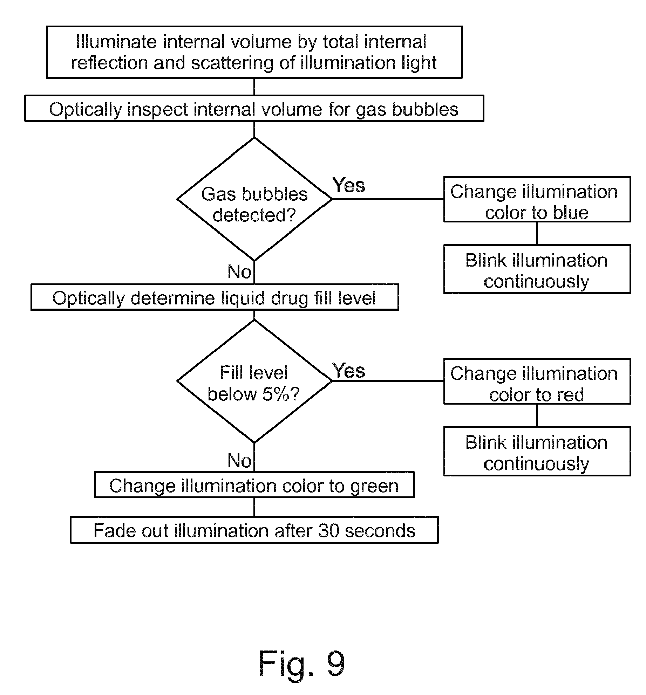

Fig. 8 shows a typical usage scenario of a liquid drug delivery system 100 as it is commonly used in form of an insulin pump during insulin infusion therapy. The drug outlet 112 of the insulin reservoir is connected to an infusion set 804 by a tubing 802, the infusion set 804 comprising a cannula for subcutaneous insertion and optionally, as shown in the Figure, an adhesive pad and a connector for the tubing 802. As described before, it may be advantageous to illuminate the tubing 802 and/or a cannula or connector of the infusion set 804 to inspect said parts for layers of fibrillated insulin, sources of obstruction, or damages. Illuminating the parts outside the drug delivery system 100 by total internal reflection may be accomplished by a dedicated light source 120 or by coupling the illumination light 600 from the reservoir into the tubing 802 and, thus, into all other parts optically connected to the tubing 802. For this purpose, all parts being in contact with the insulin should be manufactured from a material which may serve as an optical waveguide, such as the optical waveguide wall material 106 or any other optically transparent material, which is already often the case for implementations according to the state of the art. Fig. 9 shows a flowchart of an exemplary self-test routine of a liquid drug delivery system 100, where the methods disclosed for the present invention are performed as steps of the routine. The self-test starts by illuminating the internal volume 110 by total internal reflection and scattering of the illumination light 600 as a first step. In the second step, the internal volume 110 is inspected optically for gas bubbles in an automated manner by the .....??!. If gas bubbles are detected, the illumination color is changed to blue and the switching state of the light source 120 is modulated such that the user perceives a blinking illumination pattern. If no gas bubbles are detected, the procedure continues with the third step, being optically determining the liquid drug fill level in the reservoir. If the fill level is below 5% of a full reservoir charge, the illumination color is switched to red and the switching state of the light source 120 is modulated in a continuous blinking pattern. If the fill level of the reservoir is above 5%, the illumination color is switched to green and the light source 120 continues to be switched on for 30 seconds, and is faded out thereupon. As mentioned before, the optical inspection of the reservoir fill level and the presence of gas bubbles may be performed by an automatic sub-system of the liquid drug delivery system 100.

### LIST OF REFERENCE NUMERALS

- 100: Drug delivery system
- 106: Optical waveguide wall material
- 110: Internal volume
- 112: Drug outlet
- 120: Light source
- 122: Light coupling area
- 130: Drug delivery device
- 150: Pumping apparatus
- 155: piston
- 160: Light control unit
- 200: Optical coupling element
- 306: Optical waveguide support material
- 502: Optical coupling segments
- 600: Illumination light
- 602: Light scattering particles
- 700: Light scattering region
- 802: Tubing
- 804: Infusion set

## Claims

1. A drug delivery device (130) comprising:
a hollow container comprising a wall structure and an internal volume (110) surrounded by the wall structure, the hollow container being adapted to contain within or transport through the internal volume a liquid drug to be supplied to a patient, the wall structure comprising an optical waveguide wall material (106), the optical waveguide wall material comprising a light scattering characteristic; and
a light source (120), adapted to illuminate the internal volume by emitting illumination light (600) such that the illumination light is coupled into the optical waveguide wall material through a light coupling area (122) of the wall structure, propagates through the optical waveguide wall material by total internal reflection and is scattered into the internal volume by the light scattering characteristic.

2. The drug delivery device according to claim 1, further comprising an optical coupling element (200) adapted to couple the illumination light (600) through the light coupling area (122) into the optical waveguide wall material (106).

3. The drug delivery device according to claim 2, the hollow container being an injection-molded part and the optical coupling element (200) being adapted to couple the illumination light (600) into the optical waveguide wall material (106) such that the propagation direction of the illumination light entering the optical waveguide wall material is parallel to the draw direction of the injection-molded hollow container.

4. The drug delivery device according to claim 2 or 3, the light coupling area (122) being parallel to a cross-sectional wall area of the wall structure, the cross-sectional wall area being defined with respect to a radial extension of the internal volume (110).

5. The drug delivery device according to claim 4, the light coupling area (122) covering the whole cross-sectional wall area, the optical coupling element (200) being adapted to couple the illumination light (600) into the optical waveguide wall material (106) in a manner that minimizes the spatial light intensity variation across the light coupling area.

6. The drug delivery device according to claim 5, further comprising multiple ones of the light source (120), the optical coupling element comprising multiple optical coupling segments (502), each coupling segment being optically connected to at least one of the light sources and the light coupling area (122).

7. The drug delivery device according to any of the previous claims, the optical waveguide wall material (106) further comprising a light scattering region (700), the light scattering characteristic being localized in the light scattering region with a local scattering density, the local scattering density being a function of the position within the light scattering region.

8. The drug delivery device according to any of the previous claims, the hollow container further comprising a supporting structure, the supporting structure comprising an optical waveguide support material (306) and being optically connected to the optical waveguide wall material (106), the light source (120) and / or the optical coupling element (200) being adapted to couple the illumination light (600) through a light coupling area (122) of the supporting structure such that the illumination light propagates through the optical waveguide support material and the optical waveguide wall material by total internal reflection.

9. The drug delivery device according to claim 8, the light coupling area (122) being parallel to an intersectional area of the supporting structure and a cross-sectional wall area of the wall structure, the cross-sectional wall area being defined with respect to a radial extension of the internal volume (110).

10. The drug delivery device according to any of the previous claims, the hollow container further comprising a drug outlet (112) and a tubing (802), the drug outlet being in fluid connection to the internal volume (110), the tubing comprising an internal tubing volume and an optical waveguide tubing material surrounding the internal tubing volume, the tubing being adapted for extending the fluid connection from the drug outlet through the internal tubing volume to a connector or cannula of an infusion set (804) adapted to supply the liquid drug to the patient, the light coupling area (122) being a cross-sectional area of the optical waveguide tubing material with respect to a radial extension of the tubing.

11. A drug delivery system (100) comprising a drug delivery device according to any of the previous claims, the hollow container further comprising a drug outlet (112) being in fluid connection to the internal volume (110); a pumping apparatus (150) adapted for supplying the liquid drug to the patient by pressing the liquid drug through the drug outlet; and a light control unit (160) adapted for generating an electrical control signal to be transmitted to the light source (120).

12. A method of illuminating the internal volume (110) of a drug delivery system according to claim 11, the method comprising coupling the illumination light (600) into the optical waveguide wall material (106) such that the illumination light propagates through the optical waveguide wall material by total internal reflection and is scattered into the internal volume by the light scattering characteristic.

13. The method according to claim 12, further comprising optically inspecting the internal volume (110) for gas bubbles.

14. The method according to claim 12 or 13, further comprising optically determining the fill level of the liquid drug contained within the internal volume (110).

15. The method according to any of claims 12 to 14, further comprising modulating the light source (120) by the light control unit (160) for transmission of information to a user, the information being a status indication of the drug delivery device and / or system.
